# EUROPEAN PATENT SPECIFICATION

(11) **EP 4 167 847 B1**
(45) Date of publication and mention of the grant of the patent: **24.06.2026**
(21) Application number: 21829556.6
(22) Date of filing: 22.06.2021
(51) Int. Cl.: A61B 5/055, A61B 5/377, A61B 5/369

(54) **METHODS AND SYSTEMS FOR DETERMINING AND CORRECTING IMAGING ARTIFACTS**
VERFAHREN UND SYSTEME ZUR BESTIMMUNG UND KORREKTUR VON ABBILDUNGSARTEFAKTEN
PROCÉDÉS ET SYSTÈMES DE DÉTERMINATION ET DE CORRECTION D'ARTÉFACTS D'IMAGERIE

(30) Priority: 22.06.2020 US 202063042433 P
(43) Date of publication of application: 26.04.2023
(73) Proprietor: United States Government as Represented by The Department of Veterans Affairs, Washington, DC 20420 (US)
(72) Inventor: MAY, Geoffrey, Washington, DC 20420 (US)
(74) Representative: CH Kilger Anwaltspartnerschaft mbB
(86) International application number: PCT/US2021/038484
(87) International publication number: WO 2021/262720

(56) References cited:
- US-A1- 2003 081 818
- US-A1- 2012 296 195
- US-A1- 2018 055 406
- US-A1- 2018 055 406
- YAN W X ET AL: "Understanding gradient artefacts in simultaneous EEG/fMRI", NEUROIMAGE, ELSEVIER, AMSTERDAM, NL, vol. 46, no. 2, 1 June 2009 (2009-06-01), pages 459 - 471, XP026050412, ISSN: 1053-8119, [retrieved on 20090129], DOI: 10.1016/J.NEUROIMAGE.2009.01.029
- ROTHL�BBERS SVEN ET AL: "Characterisation and Reduction of the EEG Artefact Caused by the Helium Cooling Pump in the MR Environment: Validation in Epilepsy Patient Data", BRAIN TOPOGRAPHY, HUMAN SCIENCES PRESS, NEW YORK, NY, US, vol. 28, no. 2, 26 October 2014 (2014-10-26), pages 208 - 220, XP035451063, ISSN: 0896-0267, [retrieved on 20141026], DOI: 10.1007/S10548-014-0408-0

## Description

### CROSS-REFERENCE TO RELATED APPLICATION

This application claims priority to U.S. Provisional Application No.: 63/042,433, filed June 22, 2020.

### BACKGROUND

Electroencephalography ("EEG") and functional Magnetic Resonance Imaging ("fMRI" or "MRI") have complementary temporal and spatial resolutions, and promise to non-invasively inform sophisticated models of human brain activity. However, the MRI must necessarily change the strength of a magnetic field (e.g., a magnetic field generated by or otherwise associated with an MRI device) across time. Further, movement of a human body may cause movements of EEG wires that, by virtue of being disposed on a patient's head are within the magnetic field created by the MRI machine. These phenomena induce electric current, which is then measured by an EEG amplifier and shows up as an artifact in an EEG signal. Before evaluating what electrical currents are actually generated by the brain's activity, the artifact in the EEG signal generated by the MRI scanner must first be determined and corrected.

The following document relates to the modelling of gradient artefacts in simultaneous EEG and fMRI imaging : YAN W X ET AL: "Understanding gradient artefacts in simultaneous EEG/fMRI",NEUROIMAGE, ELSEVIER, AMSTERDAM, NL, vol. 46, no. 2, 1 June 2009 (2009-06-01), pages 459-471, ISSN: 1053-8119, DOI: 10.1016/ J.NEUROIMAGE.2009.01.029

### SUMMARY

The appended claims constitute a summary of the invention.

It is to be understood that both the following general description and the following detailed description are exemplary and explanatory only and are not restrictive. In an embodiment, provided are methods and systems for estimating and reducing artifacts in medical images, for instance in electroencephalography (EEG) or magnetic resonance imaging (MRI) techniques. For example, during treatment, electric signals may be recorded at one or more electrodes while magnetic field pulses are applied to a patient's head (or other body parts) by way of an MRI procedure. MRI (including both traditional MRI and function "fMRI") yields information about blood oxygenation in the human brain while in EEG, the brain creates electrical voltages while it works, which can be measured by EEG electrodes that are held on the scalp. While magnetic field pulses are repeatedly applied to the patient's head via MRI, the EEG electrodes receive continuous analog electric signals from the patient's head, which are fed to an EEG device. An amplification unit of the EEG device amplifies the continuous analog electric signals, and an analog-to-digital conversion unit of the EEG device converts the continuous analog electric signals to corresponding digital signals by sampling the continuous analog electric signals with a sampling rate which is set based on an EEG clock. The present systems and methods make use of electrodynamics to determine and reduce errors in an EEG signal caused by the MRI, then use that information to fix errors in imaging data. Provided herein is a technique which eliminates noise in both signals and thereby allows the measuring modalities to inform each other. This allows for building sophisticated models of brain activity using non-invasive technology.

Additional advantages will be set forth in part in the description which follows or may be learned by practice. The advantages will be realized and attained by means of the elements and combinations particularly pointed out in the appended claims.

### BRIEF DESCRIPTION OF THE DRAWINGS

The accompanying drawings, which are incorporated in and constitute a part of the present description serve to explain the principles of the methods and systems described herein:
**FIG. 1** shows an example system;
**FIG. 2** shows an example system;
**FIGS. 3A-3C** shows example systems and apparatuses;
**FIG. 4** shows an example diagram of tissue voxels and image pixels;
**FIG. 5** shows an example EEG gradient;
**FIG. 6** shows an example EEG gradient;
**FIG. 7** shows an example transform of fMRI images;
**FIG. 8** shows an example method; and
**FIG. 9** shows an example computing environment in which the present methods may be executed.

### DETAILED DESCRIPTION

As used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another configuration includes from the one particular value and/or to the other particular value. Similarly, when values are expressed as approximations, by use of the antecedent "about," it will be understood that the particular value forms another configuration. It will be further understood that the endpoints of each of the ranges are significant both in relation to the other endpoint, and independently of the other endpoint.

"Optional" or "optionally" means that the subsequently described event or circumstance may or may not occur, and that the description includes cases where said event or circumstance occurs and cases where it does not.

Throughout the description and claims of this specification, the word "comprise" and variations of the word, such as "comprising" and "comprises," means "including but not limited to," and is not intended to exclude, for example, other components, integers or steps. "Exemplary" means "an example of" and is not intended to convey an indication of a preferred or ideal configuration. "Such as" is not used in a restrictive sense, but for explanatory purposes.

It is understood that when combinations, subsets, interactions, groups, etc. of components are described that, while specific reference of each various individual and collective combinations and permutations of these may not be explicitly described, each is specifically contemplated and described herein. This applies to all parts of this application including, but not limited to, steps in described methods. Thus, if there are a variety of additional steps that may be performed it is understood that each of these additional steps may be performed with any specific configuration or combination of configurations of the described methods.

As will be appreciated by one skilled in the art, hardware, software, or a combination of software and hardware may be implemented. Furthermore, a computer program may exist as a product on a computer-readable storage medium (e.g., non-transitory) having processor-executable instructions (e.g., computer software) embodied in the storage medium, or as a configuration of hardware designed to implement these instructions, as in an application-specific integrated circuit. Any suitable computer-readable storage medium may be utilized including hard disks, CD-ROMs, optical storage devices, magnetic storage devices, memresistors, Non-Volatile Random Access Memory (NVRAM), flash memory, or a combination thereof.

Throughout this application reference is made to block diagrams and flowcharts. It will be understood that each block of the block diagrams and flowcharts, and combinations of blocks in the block diagrams and flowcharts, respectively, may be implemented by processor-executable instructions. These processor-executable instructions may be loaded onto a general purpose computer, special purpose computer, or other programmable data processing apparatus to produce a machine, such that the processor-executable instructions which execute on the computer or other programmable data processing apparatus create a device for implementing the functions specified in the flowchart block or blocks.

These processor-executable instructions may also be stored in a computer-readable memory that may direct a computer or other programmable data processing apparatus to function in a particular manner, such that the processor-executable instructions stored in the computer-readable memory produce an article of manufacture including processor-executable instructions for implementing the function specified in the flowchart block or blocks. The processor-executable instructions may also be loaded onto a computer or other programmable data processing apparatus to cause a series of operational steps to be performed on the computer or other programmable apparatus to produce a computer-implemented process such that the processor-executable instructions that execute on the computer or other programmable apparatus provide steps for implementing the functions specified in the flowchart block or blocks.

Blocks of the block diagrams and flowcharts support combinations of devices for performing the specified functions, combinations of steps for performing the specified functions and program instruction means for performing the specified functions. It will also be understood that each block of the block diagrams and flowcharts, and combinations of blocks in the block diagrams and flowcharts, may be implemented by special purpose hardware-based computer systems that perform the specified functions or steps, or combinations of special purpose hardware and computer instructions.

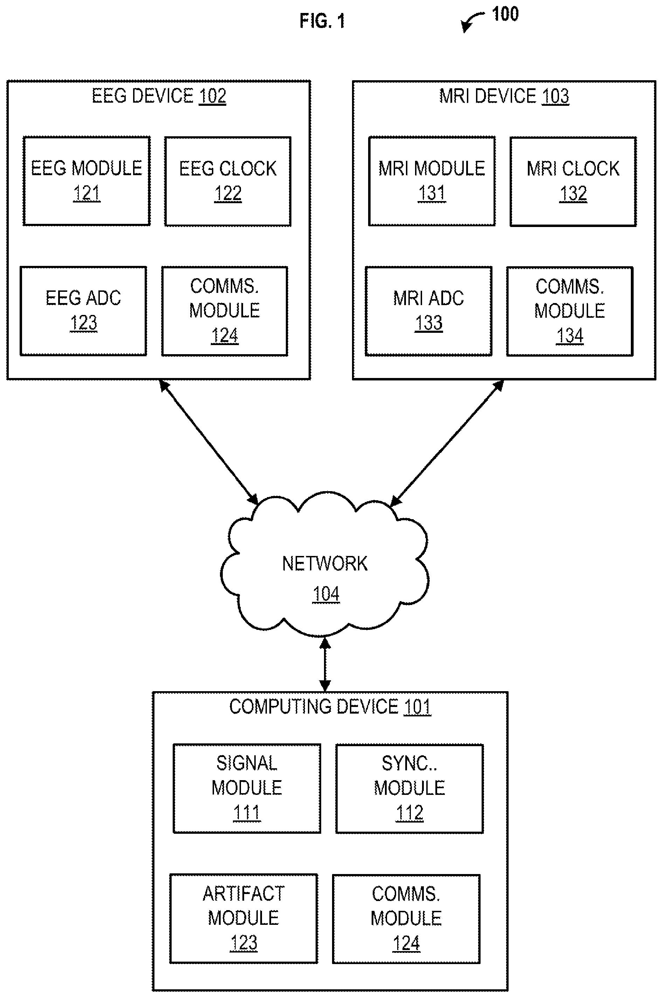

**FIG. 1** shows an example system **100.** The system **100** may comprise a computing device **101,** an EEG device **102,** an MRI device **103,** and a network **104.** The computing device **101** may comprise for example, a smart phone, a tablet, a laptop, a desktop computer, a server, combinations thereof, and the like. The computing device **101** may send and receive data to/from the EEG device **102** and/or the MRI device **103** via the network **105.** The network **105** may be an optical fiber network, a coaxial cable network, a hybrid fiber-coaxial network, a wireless network, a satellite system, a direct broadcast system, a local network, a wide area network, a public network, a private network, combinations thereof, and the like. The network **105** can be the Internet.

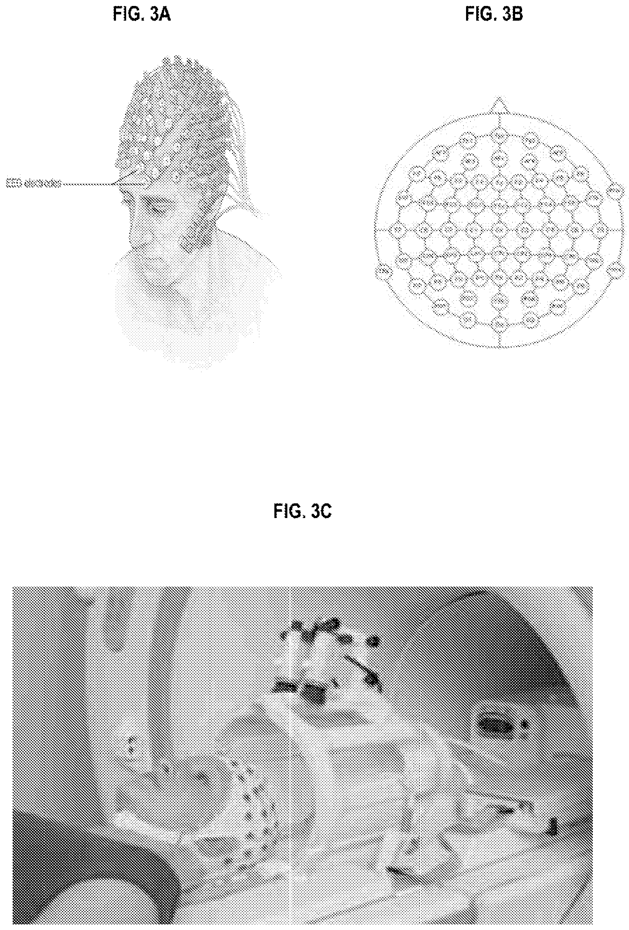

The EEG device **102** may comprise an EEG module **121,** an EEG clock **122,** an EEG analog to digital converter (EEG ADC) **123,** and a communications module **124.** The EEG device **102** may be configured to administer an EEG test (e.g., an EEG procedure). The EEG device **102** may comprise one or more electrodes (e.g., EEG electrodes). For example, the EEG module **121** may comprise the one or more electrodes and any electrical connection therewith (e.g., between various components of the system). Each electrode of the one or more EEG electrodes is associated with a location (e.g., a position). During the EEG procedure, the one or more electrodes may be placed near a patient's head. For example, the one or more electrodes may be disposed directly on the scalp of the patient or the one or more electrodes may be disposed on a wearable cap configured to be placed on the head of the patient as seen in **FIGS. 3A-3C** and described in more detail below. The one or more electrodes may be configured to detect one or more changes in one or more electrical signals (e.g., one or more voltages, one or more charges, etc.) which may result from activity within the brain of the patient. Based on the one or more voltages or charges, the EEG module **121** may be configured to generate and/or otherwise determine one or more EEG waveforms. Each EEG waveform of the one or more EEG waveforms may be associated an electrode of the one or more electrodes. For example, an EEG waveform for the one or more EEG waveforms may be associated with an electrode of the one or more electrodes. The EEG waveform may comprise an analog signal (e.g., an EEG waveform analog signal). The EEG module may comprise an EEG amplifier. The EEG amplifier may be configured to amplify the one or more changes in the one or more charges. The EEG module **121** may send the EEG waveform analog signal to the EEG ADC **123.** The EEG ADC **123** may be configured to receive the EEG waveform analog signal and convert receive the EEG waveform analog signal to an EEG waveform digital signal. The system **100** may perform precise computation of EEG electrodes' position in space, as well as the shape of the magnetic field they inhabit, as both of these physical quantities evolve over time. An iterative process combines knowledge of a typical echo planar imaging sequence and the moment-to-moment changes in EEG voltage measured to progressively solve for unknown quantities until information can be used to enhance the fidelity of both EEG and MRI signals.

The MRI device **103** may comprise an MRI module **131,** an MRI clock **132,** an MRI analog to digital converter (MRI ADC) **133,** and a communications module **134.** The MRI device **103** may be configured to administer an MRI test (e.g., an MRI procedure). The MRI device 103 may be configured to generate a magnetic field. By virtue of the magnetic field, the MRI device **103** may be configured to cause atomic nuclei to spin and emit radio waves in a systematic way, such that every voxel in a slice is encoded with a unique combination of frequency and phase. The MRI device **103** may apply a "gradient," configured to generate a magnetic field which is weaker in some places and stronger in others. A person skilled in the art will appreciate imaging quality is increased when the gradient (e.g., from a weak field strength to a strong field strength) is linear. The shape of the actual gradient may be determined by acquiring a field map before scanning starts. A gradient change over time and an interplay with spatial heterogeneity in magnetic susceptibility can be determined and thus can reduce distortions imparted to the MRI images.

The MRI device **103** may comprise one or more magnets. For example, the MRI module **131** may comprise the one or more magnets and any electrical connection therewith (e.g., between various components of the system). During the MRI procedure, the one or more magnets may be placed near a patient's head. The one or more magnetics may be configured to generate the magnetic field. The magnetic field may be configured to impart an electromagnetic force on charged particles (e.g., by virtue of their presence in the magnetic field) in the brain of the patient (e.g., a proton associated with, for example, a hydrogen atom, an oxygen atom, etc.). The electromagnetic force associated with the magnetic field may cause the charged particle to align itself (e.g., in terms of alignment, phase, frequency, spin, direction, combinations thereof, and the like) in a direction associated with a gradient in a magnetic field generated by the MRI device **103.** The MRI device **103** may be configured to generate a radio frequency pulse which may cause the charged particle to temporarily deviate from the alignment with the magnetic field, and, after the radio frequency pulse ends, "snap" back into alignment (e.g., the charged particle may "realign" with the magnetic field). During the realignment of the charged particle (e.g., the proton) with the magnetic field, energy may be released. This released energy may indicate oxygen levels (by virtue of a quantity of protons associated with hydrogen and oxygen) in various regions of the brain (as determined by taking volumetric "slices" and compiling the volumetric slices). The oxygen levels may be used to generate an MRI image which may indicate areas of higher and lower oxygenation, thereby generating an MRI "map" of the brain. The MRI module **131** may be configured to detect or otherwise determine blood oxygen levels in or more regions of the brain. The MRI module **131** may be configured to generate and/or otherwise determine an MRI waveform. The MRI waveform may comprise an analog signal (e.g., an MRI waveform analog signal). The MRI module **131** may send the MRI waveform analog signal to the MRI ADC **133.** The EEG ADC **123** may be configured to receive the MRI waveform analog signal and convert the MRI analog waveform signal to an MRI waveform digital signal.

The computing device **101** may comprise a signal processing module **111,** a synchronization module **112,** an artifact module **113,** and a communications module **114.** The computing device **101** may be configured to send and receive data. For example, the computing device **101** may comprise a communications module **114** configured to send and receive data. The communications module **114** may be configured to send and receive data to and/or from one or more other devices including, for example, the EEG device **102** and/or the MRI device **103.**

The signal processing module **111** may be configured to receive, either directly or via the communications module **104,** one or more signals. The one or more signals may comprise, for example, one or more analog signals and/or one or more digital signals. The one or more signals may comprise one or more EEG signals and/or one or more MRI signals. The signal processing module may be configured to convert the one or more analog signals to one or more digital signals and vice versa. The signal processing module **111** may be configured to process the signals according to known techniques including by processing the one or more signals based on frequency, phase, amplitude, magnitude, combinations thereof, and the like. The signal processing module **111** may be configured to apply to a signal of the one or more signals, one or more filters including low-pass filters, high-pass filters, noise reduction filters, combinations thereof, and the like. The signal processing module **111** may receive or otherwise determine one or more EEG signals. The one or more EEG signals may be received from the EEG device. The one or more EEG signals may comprise one or more digital signals and/or one or more analog signals. The signal processing module **111** may receive the one or more MRI signals. The one or more MRI signals may comprise digital signals or analog signals.

The computing device **101** may receive EEG timing data from the EEG device **102.** Similarly, the computing device **101** may receive MRI timing data from the MRI device **103.** The synchronization module **112** may be configured to receive the EEG timing data from the EEG device **102** and the MRI timing data from the MRI device **103.** The synchronization module **112** may be configured to synchronize EEG data and MRI data based on the EEG timing data and the MRI timing data. For example, the synchronization module **112** may determine EEG timing data associated with one or more EEG readings. For example, the synchronization module **112** may determine MRI timing data associated with one or more MRI pulses (e.g., electromagnetic pulses used in MRI and fMRI procedures). Based on the respective timing data, the computing device **101** may be configured to determine one or more MRI scanning slices associated with the MRI. They synchronization module **112** may be configured to control either the EEG clock **122** and/or the MRI clock **132** (and therefore the EEG device **102** and the MRI device **103)** by way of a phase locked loop circuitry to synchronize the EEG clock **122** and the MRI clock **132** to coordinate EEG readings and MRI pulses. Thus, the one or more electromagnetic pulses associated with the MRI may be temporally associated with EEG readings. The one or more electromagnetic pulses associated with the MRI may be temporally associated with readings from other devices such as a respiratory belt or heart rate monitor. The current manifestation uses technology in which the timing of EEG voltage digitization is synchronized with electrical synchronization pulses emitted by the MRI scanner to the EEG amplifier hardware. This ensures maximum similarity from one brain volume to the next, but is not strictly necessary as other methods such as upsampling and cubic interpolation could be used to align one MRI slice to the next and one MRI volume to the next. While the standard method takes a moving average of the imparted gradient artifact, the proposed method extracts information about both electrode and connected wire positions and velocities imparted by both head movement and blood vessel expansion and contraction, as well as change in magnetic field strength caused by the displacement of water with air during respiration.

The MRI timing data may indicate one or more scanning slices. For example, a scanning slice may be associated with a timeframe (e.g., 40 milliseconds). Individually and/or collectively, the one or more MRI scanning slices may comprise or be associated with one or more MRI scanning slice parameters The one or more MRI scanning slices parameters may comprise, for example, one or more MRI magnetic field characteristics (e.g., strength, shape, gradient, etc., combinations thereof and the like). A person skilled in the art will appreciate the aforementioned characteristics are merely exemplary and explanatory. For example, the computing device **101** may be configured to determine where a first MRI scanning slice of the one or more MRI scanning slices ends and a second MRI scanning slice of the one or more MRI scanning slices begins. For example, The MRI clock may generate or otherwise determine an MRI pulse every 3.2 seconds. Based on the MRI pulse, it may determined that the MRI device **103** has started a new scan on the entire volume of the brain. The scan may be is subdivided into slices (e.g., 40 slices each 80 milliseconds long). Correlations between slice length and timing data may be determined manually, learned over time, and/or input by an operator. A maximum correlation may be determined to indicate an ideal slice length.

The MRI device **103** and/or the computing device **101** may be configured to determine when an MRI magnetic field gradient begins and ends. For example, the computing device **101** may determine that a voltage measured in the EEG amplifier may vary linearly with respect to a current travelling through an EEG wire (e.g., to or from an EEG electrode). Based on the laws of electrodynamics, the computing device **101** may determine the derivative of the current with respect time indicates a flux associated with the MRI magnetic field. The computing device **101** determines a position associated with each electrode of the one or more electrodes. For example, the position may be determined based on a model of the head of the patient and/or input by an operator. The position may comprise x, y, and z coordinates (e.g., Cartesian coordinates in 3-D space, however any coordinate system such as Euclidean and non-Euclidean coordinates systems may be used). Thus, by knowing the position of each electrode, the current at the electrode (and derivative thereof), a flux associated with the position of the electrode within the magnetic field is determined. A diagram showing the layout of electrodes during EEG is shown in **FIG. 3B** and discussed further below. By plotting the flux and coordinates, a gradient map can be determined. Based on the position of each electrode of the one or more of electrodes, and the above described determination of the magnetic field flux associated with the MRI magnetic field determined on a per-electrode basis, an MRI magnetic field map may be determined which may indicate a flux of the MRI magnetic field at each EEG electrode. Therefore, a beginning of the gradient may be determined and an end of the gradient may be determined.

The MRI gradient may impart large changes in the EEG signal that are much larger than all other measured signals and thus easily detected. By assuming that voltage in the EEG amplifier varies linearly with the current traveling through the wire, and that the derivative of the current with respect to time estimates the change in magnetic flux, the approximate spatial location of the EEG electrodes and wires can be determined based on the current induced by the gradient. This can be combined with a-priori knowledge of the electrodes' and wires' locations in either a spherical head model, or a previously constructed 3-D model of the subject's head and electrode locations, using a method such as photogrammetry (e.g. Localite) or sterotaxy via electromagnetic receiver probe (e.g. Polhemus). Thus, the MRI's magnetic pulses influence on voltage measured at each electrode may be determined. Thus, the EEG waveform can be processed to remove phantom voltages imparted at an electrode by the MRI process which in turn can be used to correct imaging errors.

The computing device **101** may be configured to determine one or more vectors (e.g., an eigenvector, a tensor, etc., combinations thereof and the like). For example, the computing device **101** may determine an electric current associated with the EEG device **102** (e.g., with each electrode of the one or more electrodes). The electric current associated with the EEG device **102** may alternate it's current between a phase-encoding direction (e.g., front to back) and a frequency encoding direction (e.g., left to right). Thus, based on a model of the head of the patient (whether spherical or any other shape), the computing device **101** may determine a spatial organization vector (e.g., x, y, z, and flux) at each EEG electrode. The spatial organization vector may comprise a voxel. The voxel may be determined across a time domain for every EEG sample at each EEG electrode. The voxel may be computed for each point in time (for each of 5000 EEG samples per second) using principal component analysis (PCA) where each observation (i.e. eigenvector) consists of an EEG electrode's x, y, z, coordinates and estimated flux based on the time derivative of the measured current. The first eigenvector of this PCA analysis yields has (x, y, z, flux) coordinates, and describes >95% of the signal during dephase gradients, and can be normalized such that (x, y, z) is length 1. After normalization, the flux component of the eigenvector then represents an estimation of the strength of the gradient field, which varies over time with respiration and is also impacted by movement. The estimated magnetic field strength may vary with the intensity of the MRI global signal. The estimated magnetic field strength may vary over the course of a single run of scanning to track with drift as the superconducting magnetic coil loses electrical current. Thus, an MRI artifact in an EEG signal may be determined and removed. For example, the subtle ways in which the alignment of the normalized spatial (x, y, z) vector changes over time to track with rotational head motion parameters and/or respiration that are computed from the MRI image after it is reconstructed. The alignment changes may indicate the MRI artifact and correcting for the alignment changes may reduce the MRI artifact. The alignment of each estimated electrode location can be maximized via a process of gradient descent. These "virtual" electrode locations may be further refined by adding electrically connected bits of wire until a model with maximum explanatory power is arrived at which matches the digitally measured electrode locations.

The magnetic flux that EEG wires detect may originate mainly from the switching of the MRI scanner gradients themselves. The present systems and methods may make use of a tensor that describes the direction that each voxel in the MRI magnetic field is pointing, and the rate of precession based on the strength of the magnetic field. Based on the laws of electrodynamics, a voxel of the MRI magnetic field will impart more flux on an EEG wire when the MRI magnetic fields are closer to the wire (inversely proportional to the cube of the distance), more aligned with one another, precessing more quickly due to stronger gradients, or aligned in plane with the wire, having more surface area through which magnetic field lines can pass. The apparent scrambling of the MRI magnetic field achieved by a dephasing gradient reduces an MRI magnetic field voxel's alignment with its neighbors, which explains the rapid reduction in flux measured by the EEG. Further, it may be determined that, based on induced currents being in opposition to a reference electrode, slower precession is associated with an apparent "negative" voltage.

When analyzing a single slice, these gradients may describe primarily where the electrodes are located in phase encoding direction (e.g. front to back) during the slice selection gradient, and the frequency encoding direction (e.g. left to right) during the dephase gradient. This provides X and Y coordinates of electrode location estimations that can be recomputed once every slice, (e.g. every 80 milliseconds). An additional slice height, or Z coordinate, can be estimated based on variations in the electrode's characteristic response to slice level, and thus, the Z coordinate may be determined.

The PCA may be computed again, this time including the estimated change in flux at each time point for one or more channels, flattened into a row, one row for each slice of the volume. The change in flux may correspond to a change in position of the electrode. A first component of the PCA may indicate a symmetrical biphasic signal almost exclusively in the dephase gradient portion of the scan, with greater participation of electrodes with low Z values (toward the feet), which decreases down to negative participation of electrodes with high Z values (toward the superior aspect of the head). The eigenvalues across the 40 slices of the volume follow an interlacing sequence which matches the one specified by the MRI manufacturer; this fact may be used to verify the computation was carried out correctly.

With (x, y, z) coordinates for each electrode, it may be expected that only rigid body transforms of rotation and translation should apply as head movement occurs. Thus, image distortions may be determined. For example, scaling distortions may occur in the phase-encoding direction (e.g., front to back), and skew distortions in the frequency-encoding direction (e.g., left to right). Scaling distortions from front to back may correlate with the patient's degree of lung inflation, which may be measured using an MRI-safe electronic respiration belt. With current image-processing technology, false motion parameters are introduced by this phase scaling distortion which tracks with that of breathing. These distortions may be temporally associated with respiratory timing data, thereby identified in the EEG signal, and thus elimination of these distortions and improved image quality may be achieved.

The artifact module may be configured to determine one or more signal artifacts (e.g., one or more image artifacts). During the excitation of a single fMRI slice, the time derivative of the electric current induced by the gradient field alternates its organization between a phase-encoding direction (front-to-back) and the frequency-encoding direction (left-to-right). Beginning with a basic spherical head model, there exists during the application of dephasing gradients a strict spatial organization that can be summarized by a single vector. The one or more image artifacts may be associated with the scaling distortion or skew distortion as described above. The one or more signal artifacts may comprise one or more EEG signal artifacts and one or more MRI artifacts.

In an embodiment, enhancement or "unwarping" of the fMRI image may be achieved by correcting for the interaction between the temporal variation in magnetic field strength induced by respiration and the spatial heterogeneity in magnetic susceptibility that is inherent to the human head. For example, a difference between a slice at Time 1 and a second slice from the same height at Time 2 may be determined. A grid of one or more control points can be overlaid on the slice, and each control point can independently apply X and Y translations to the data in the underlying slice. For example, the translation of each voxel in the slice may be interpolated between control points with bicubic interpolation. The density of the grid of one or more control points may be increased to arbitrary precision. By minimizing a cost function such as the squared difference between the two slices, the optimal position of each control point can be computed by gradient descent. When each control point of the one or more control points is weighted for its effect on the amount of T2* signal translated, a PCA of these weighted positions may be applied to obtain, for example, two eigenvectors which, in the absence of head motion, account for a large proportion of the variation in optimal control point location over the course of time. The eigenvalues of these weighted control point position components can be shown to correspond with deep and shallow breathing by comparing with a respiration belt or the variation in global signal strength from slice to slice. In the presence of head motion, rather than matching one slice directly to another, rotation parameters derived from the EEG can be used to fit the acquired T2* slice onto a previously acquired anatomical T2 whole brain volume, with the slice appropriately rotated and translated in space, although the gradient descent procedure could also allow the slice orientation and translation perpendicular to the slice plane to move relative to the T2 volume as additional parameters to be optimized. A representative weighted average of the higher resolution T2 image can be used to test goodness of fit to a lower resolution T2* fMRI slice voxel. The "best guess" temporal variation of cerebral blood flow may be iteratively incorporated in the model to minimize the error in translation induced by actual signal variation.

Field maps, (e.g. blip-up blip-down maps in the phase encoding direction) may may be implemented in order to characterize spatial heterogeneity of the head and brain so that a gradient descent process can be constrained or enhanced. The T2* field maps, acquired in opposing directions, can be compared to each other or to the T2 signal.

Estimation of electrode location may also be utilized to facilitate removal of a ballistocardiogram artifact. The ballistocardiogram artifact may be introduced into EEG during fMRI scanning. The ballistocardoigram may be associated with an expansion and contraction of blood vessels in the scalp which may cause the one or more EEG electrodes and wires to move in the MRI magnetic field, again inducing a measured electrical current that does not reflect brain activity. Similar to accepted methods, electrocardiogram may be acquired along with EEG and rhythmic heartbeat waves (e.g., "R waves") may be identified in the time series, with a method such as wavelet convolution.

Each electrode of the one or more electrodes may have a stereotyped movement that is identified in the vicinity of each R wave (e.g., a slight expansion in volume of the temples during a heartbeat similar to how a pulse may be measured by feeling a patient's wrist), with time offsets to represent an arterial and venous latencies and a triphasic waveform that is fit to the data. These parameters may be informed by arteriography, venography, and atherosclerosis inferred from subject age, body mass index, and other factors. The effect of movement through the magnetic field on induced voltage may change depending on whether or not gradient fields are active during the period of movement.

The temporal variation in cardiac contractility may be modeled to change from heartbeat to heartbeat as a function of left ventricular filling according to the Frank-Starling mechanism. This degree of filling may be estimated using intrathoracic pressure inferred from measured or modeled respiratory phase, using either the previously described derivations as well as the R-R interval of the EKG.

The degree of electrode movement may also be influenced by trends in contractility caused by changes in autonomic tone, which may be estimated using measured pupil dilation and galvanic skin response.

These methods may apply to the use of EEG for measuring, recording, and processing electrical voltages as they evolve over time. Measuring EEG during an MRI scan carries some risk because the powerful gradient fields can produce heat that may harm the subject or damage the equipment. This necessitates the time-consuming task of applying gel to the subject's scalp to achieve sufficiently low impedances before scanning starts. These requirements may be alleviated by a multi-channel antenna with conductive probes that extend into the space in the immediate vicinity of the subject's head, without establishing electrical contact. Moreover the wires leading to these antenna probes may be shielded except in the specific locations and orientations where magnetic field density is known to vary the most, such as front to back in the phase-encoding direction. The shielding may also reduce the electrical energy imparted to the antenna by the gradient fields, reducing the risk to safety.

The method for correction of fMRI data can be performed in the complete absence of any electrical field measurements. Although the inferred electrode location provides constraints on the search space, the slice-by-slice nonlinear correction has been applied to fMRI data with the complete absence of EEG data. While computationally intensive and sensitive to head motion, this method could be applied retrospectively to improve existing fMRI data.

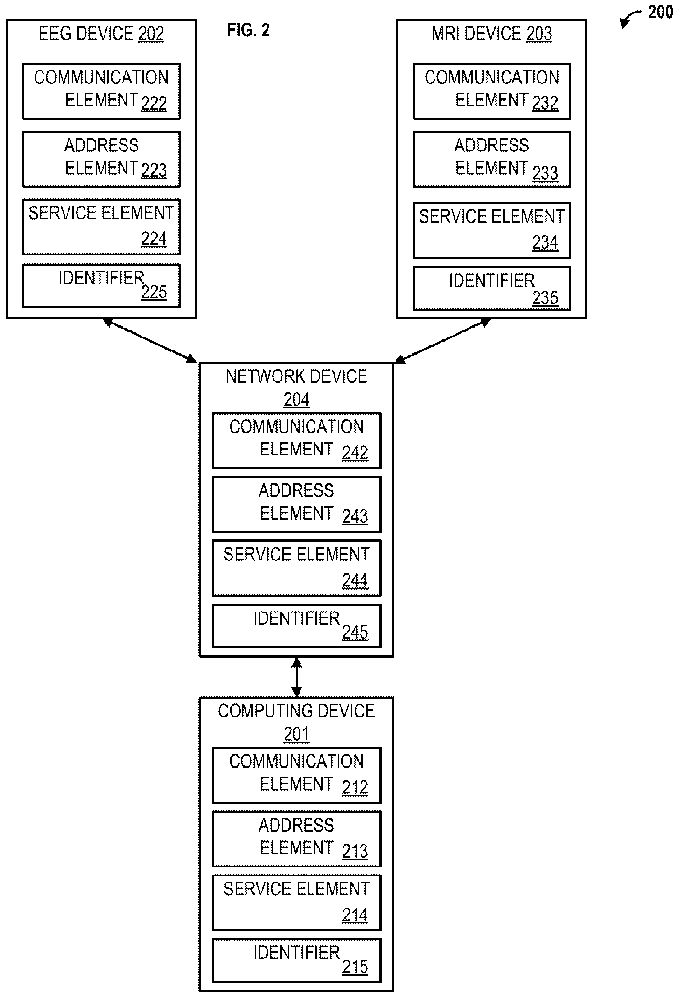

**FIG. 2** shows an example system **200.** Those skilled in the art will appreciate that digital equipment and/or analog equipment may be employed. One skilled in the art will appreciate that provided herein is a functional description and that the respective functions may be performed by software, hardware, or a combination of software and hardware.

The system **200** may comprise a computing device **201** (e.g., the computing device **101),** an EEG device **202** (e.g., the EEG device **102),** an MRI device **203** (e.g., the MRI device **103),** and a network device **204.** The computing device **201,** the EEG device **202,** and the MRI device **203** may each be configured to communicate via the network device **212.** The network device **212** may be configured to facilitate communications via a network (e.g., the network **105).** The network device **212** may be configured as a local area network (LAN). The network device **212** may be a dual band wireless communication device. The network device **212** may be a gateway device for communicating with another network, such as a communication network provided by an Internet Service Provider. The network device **212** may be configured with a first service set identifier (SSID) (e.g., associated with a user network or private network) to function as a local network for a particular user or users. The network device **212** may be configured with a second service set identifier (SSID) (e.g., associated with a public/community network or a hidden network) to function as a secondary network or redundant network for connected communication devices. The network device **212** may be configured to allow one or more wireless devices to connect to a wired and/or wireless network using Wi-Fi, Bluetooth, ZigBee, Z-Wave, or any desired method or standard.

The network device **204** may have a communication element **242,** an address element **243,** a service element **244,** an identifier **245.** The communication element **242** may comprise one or more wireless transceivers configured to transmit and receive wireless communications via a wireless communication network (e.g., the network **105).** The communication element **242** may be configured to communicate via a specific network protocol for example, a Wi-Fi protocol and/or a Wireless Personal Area Network (WPAN) protocol. The WPAN may operate using a low-energy network protocol, such as a ZigBee network protocol; a Bluetooth^{™} network protocol, a Z-Wave network protocol, a combination thereof, and/or the like.

The computing device **201** may comprise a communication element **212.** The communication element **212** may be configured to communicate with communication element **242** of the network device **106.** The communication elements **212** and **242** may be configured to communicate via a specific network protocol. The communication element **212** may be a wireless transceiver configured to communicate via a Wi-Fi network.

The computing device **201** may comprise an address element **213.** The address element **213** may be, for example, an internet protocol address, a network address, a media access control ("MAC") address, an Internet address, or the like. The address element **213** may be relied upon to establish a communication session between the computing device **201** and the network device **204** or other devices and/or networks (e.g., the EEG device **202** and the MRI device **203).** The address element **213** may be used as an identifier or locator of the control module **201.** The address element **213** may be persistent for a particular network.

The computing device **201** may comprise a service element **214.** The service element **214** may comprise an identification of a service provider or other organization (e.g., a company) associated with the computing device **201.** The class of the computing device **201** may be related to a type of device, capability of device, type of service being provided, and/or a level of service. The level of service may be, for example, business class, service tier, service package a combination thereof; and/or the like. The service element **214** may comprise information relating to or provided by a communication service provider (e.g., Internet service provider) that is providing or enabling data flow such as communication services to the computing device **201.** The service element **214** may comprise information relating to a preferred service provider for one or more particular services relating to the computing device **201.** The address element **203** may be used to identify or retrieve data from/via the service element **214,** or vice versa. One or more of the address element **213** or the service element **214** may be stored remotely from the computing device **201.** Other information may be represented by the service element **214.**

The computing device **201** may comprise an identifier **215.** The identifier **215** may be or relate to, for example, an Internet Protocol (IP) Address IPV4/IPV6 or a media access control address (MAC address) or the like. The identifier **215** may be a unique identifier for facilitating wired and/or wireless communications with the network device **204.** The identifier **215** may be associated with a physical location of the computing device **201** and/or the network device **204.**

The EEG device **202** may comprise a communication element **222.** The communication element **222** may be configured to communicate with communication element **242** of the network device **106.** The communication elements **222** and **242** may be configured to communicate via a specific network protocol. The communication element **222** may be a wireless transceiver configured to communicate via a Wi-Fi network.

The EEG device **202** may comprise an address element **223.** The address element **223** may be, for example, an internet protocol address, a network address, a media access control ("MAC") address, an Internet address, or the like. The address element **223** may be relied upon to establish a communication session between the EEG device **202** and the network device **204** or other devices and/or networks (e.g., the computing device **201** and/or the MRI device **203).** The address element **223** may be used as an identifier or locator of the EEG device **202.** The address element **223** may be persistent for a particular network.

The EEG device **202** may comprise a service element **224.** The service element **224** may comprise an identification of a service provider or other organization (e.g., a company) associated with the EEG device **202.** The class of the EEG device **202** may be related to a type of device, capability of device, type of service being provided, and/or a level of service. The level of service may be, for example, business class, service tier, service package a combination thereof; and/or the like. The service element **224** may comprise information relating to or provided by a communication service provider (e.g., Internet service provider) that is providing or enabling data flow such as communication services to the EEG device **202.** The service element **224** may comprise information relating to a preferred service provider for one or more particular services relating to the EEG device **202.** The address element **223** may be used to identify or retrieve data from/via the service element **224,** or vice versa. One or more of the address element **223** or the service element **224** may be stored remotely from the computing device **201.** Other information may be represented by the service element **224.**

The EEG device **202** may comprise an identifier **225.** The identifier **225** may be or relate to, for example, an Internet Protocol (IP) Address IPV4/IPV6 or a media access control address (MAC address) or the like. The identifier **225** may be a unique identifier for facilitating wired and/or wireless communications with the network device **224.** The identifier **215** may be associated with a physical location of the EEG device **202.**

The MRI device **203** may comprise a communication element **232.** The communication element **232** may be configured to communicate with communication element **242** of the network device **106.** The communication elements **232** and **242** may be configured to communicate via a specific network protocol. The communication element **232** may be a wireless transceiver configured to communicate via a Wi-Fi network.

The MRI device **203** may comprise an address element **233.** The address element **233** may be, for example, an internet protocol address, a network address, a media access control ("MAC") address, an Internet address, or the like. The address element **233** may be relied upon to establish a communication session between the MRI device **203** and the network device **204** or other devices and/or networks (e.g., the computing device **201** and/or the EEG device **202).** The address element **233** may be used as an identifier or locator of the MRI device **203.** The address element **233** may be persistent for a particular network.

The MRI device **203** may comprise a service element **234.** The service element **234** may comprise an identification of a service provider or other organization (e.g., a company) associated with the MRI device **203.** The class of the MRI device **203** may be related to a type of device, capability of device, type of service being provided, and/or a level of service. The level of service may be, for example, business class, service tier, service package a combination thereof; and/or the like. The service element **234** may comprise information relating to or provided by a communication service provider (e.g., Internet service provider) that is providing or enabling data flow such as communication services to the MRI device **203.** The service element **234** may comprise information relating to a preferred service provider for one or more particular services relating to the MRI device **203.** The address element **234** may be used to identify or retrieve data from/via the service element **234,** or vice versa. One or more of the address element **223** or the service element **234** may be stored remotely from the computing device **201.** Other information may be represented by the service element **234.**

The MRI device **203** may comprise an identifier **235.** The identifier **235** may be or relate to, for example, an Internet Protocol (IP) Address IPV4/IPV6 or a media access control address (MAC address) or the like. The identifier **235** may be a unique identifier for facilitating wired and/or wireless communications with the network device **204.** The identifier **235** may be associated with a physical location of the MRI device **203.**

**FIG. 3A** shows an exemplary arrangement of a plurality of EEG electrodes and wires. The arrangement of EEG electrodes may be associated with the EEG formation data. The EEG formation data may comprise data indicating a position in space for each EEG electrode of the plurality of EEG electrodes. For instance, the EEG formation data may indicate where each of the EEG electrodes is located in a 3D coordinate grid (e.g., and xyz-volumetric grid). Each EEG electrode may be associated with a coordinate triplet. The coordinate triplet may comprise an x-coordinate, a y-coordinate, and a z-coordinate (x₁, y₁, z₁). The plurality of EEG electrodes may be placed in a magnetic field, for instance the magnetic field associated with an MRI imaging technique as executed by an MRI machine. **FIG. 3B** shows an exemplary EEG electrode diagram. **FIG. 3C** shows an exemplary system used for simultaneous EEG/MRI.

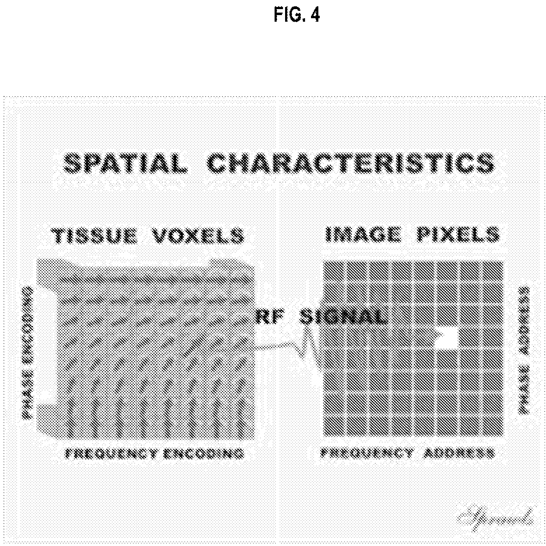

**FIG. 4** shows an exemplary illustration of tissue voxels and image pixels. A plurality of tissue voxel may be determined. The plurality of tissue voxels may comprise a plurality of tissue volumes over time. The plurality of tissue voxels may be associated with a plurality of tensors. The plurality of tensors may indicate a frequency and a phase associated with each voxel. The plurality of tensors may be associated with a plurality of signals, for example electromagnetic signals such as radio frequency signals. The plurality of signal may be converted, through any known image processing technique into pixels representing any of the EEG image data, the MRI image data, or the combined image data. The MRI device may configured to cause atomic nuclei to spin and emit radio waves in a systematic way, such that every voxel in a slice is encoded with a unique combination of frequency and phase. The present systems and methods may make use of a tensor that describes the direction that each voxel in the MRI magnetic field is pointing, and the rate of precession based on the strength of the magnetic field. Based on the laws of electrodynamics, a voxel of the MRI magnetic field will impart more flux on an EEG wire when the MRI magnetic fields are closer to the wire (inversely proportional to the cube of the distance), more aligned with one another, precessing more quickly due to stronger gradients, or aligned in plane with the wire, having more surface area through which magnetic field lines can pass. The apparent scrambling of the MRI magnetic field achieved by a dephasing gradient reduces an MRI magnetic field voxel's alignment with its neighbors, which explains the rapid reduction in flux measured by the EEG. Further, it may be determined that, based on induced currents being in opposition to a reference electrode, slower precession is associated with an apparent "negative" voltage. The computing device **101** may be configured to determine one or more vectors (e.g., an eigenvector, a tensor, etc., combinations thereof and the like). For example, the computing device **101** may determine an electric current associated with the EEG device **102.** The electric current associated with the EEG device **102** may alternate it's current between a phase-encoding direction (e.g., front to back) and a frequency encoding direction (e.g., left to right). Thus, based on a model of the head of the patient (whether spherical or any other shape), the computing device **101** may determine a special organization vector (e.g., x, y, z, flux). The spatial organization vector may comprise a voxel. The spatial organization voxel may be determined across a time domain for every EEG sample at each EEG electrode.

When analyzing a single slice, these gradients may describe primarily where the electrodes are located in phase encoding direction (e.g. front to back) during the slice selection gradient, and the frequency encoding direction (e.g. left to right) during the dephase gradient. This provides x and y coordinates of electrode location estimations that can be recomputed once every slice, (e.g. every 80 milliseconds). An additional slice height, or Z coordinate, can be estimated based on variations in the electrode's characteristic response to slice level, and thus, the Z coordinate may be determined.

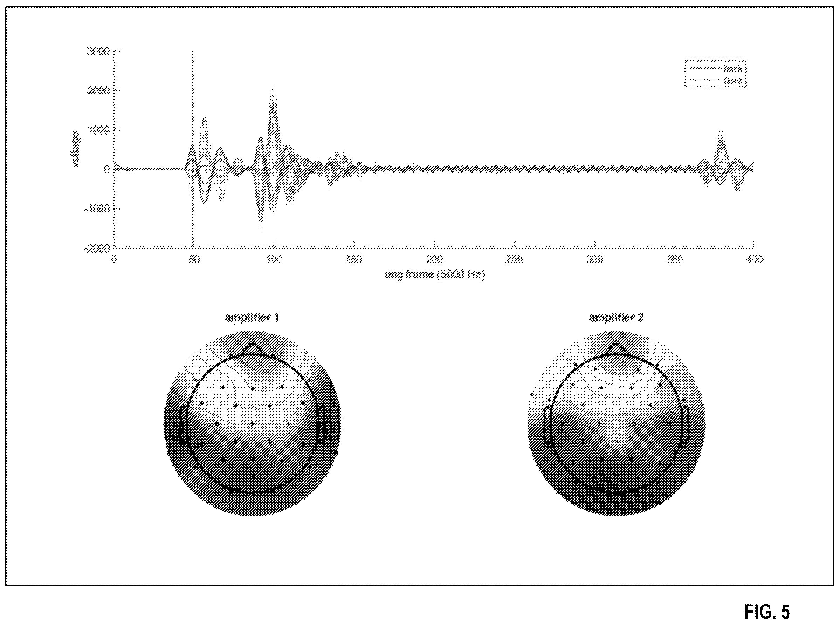

**FIG. 5** shows an example EEG gradient during a single slice of excitation during echo planar imaging. Each line represents the voltage at one electrode, with posterior electrodes shaded darker and anterior electrodes shaded lighter. A line is drawn during a putative slice selection gradient period just prior to 50^{th} EEG frame and the topological layout of the voltages is illustrated (below). 64 EEG channels are divided among two amplifier boxes, as the separate paths taken by their ribbon cables confounds an otherwise orderly spatial organization. The aforementioned example is meant to be exemplary and explanatory only and is not intended to be limiting. For instance, the EEG gradient may be displayed in a colorized manner with associations between respective hues and electrodes.

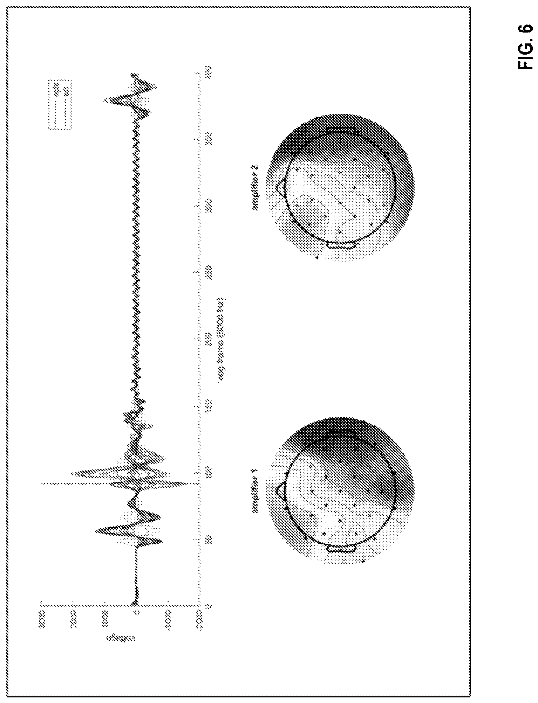

**FIG. 6** shows an EEG gradient during a single slice of excitation during echo planar imaging. Each line represents the voltage at one electrode, with electrodes on the left shaded lighter and electrodes on the right in darker shades. A line is drawn during a putative dephase gradient just prior to 100^{th} EEG frame and the topological layout of the voltages at that timepoint is illustrated (below). 64 voltage channels may be divided among two amplifier boxes, as the separate paths taken by their ribbon cables confounds an otherwise orderly spatial organization. The aforementioned example is meant to be exemplary and explanatory only and is not intended to be limiting. For instance, the EEG gradient may be displayed in a colorized manner with associations between respective hues and electrodes.

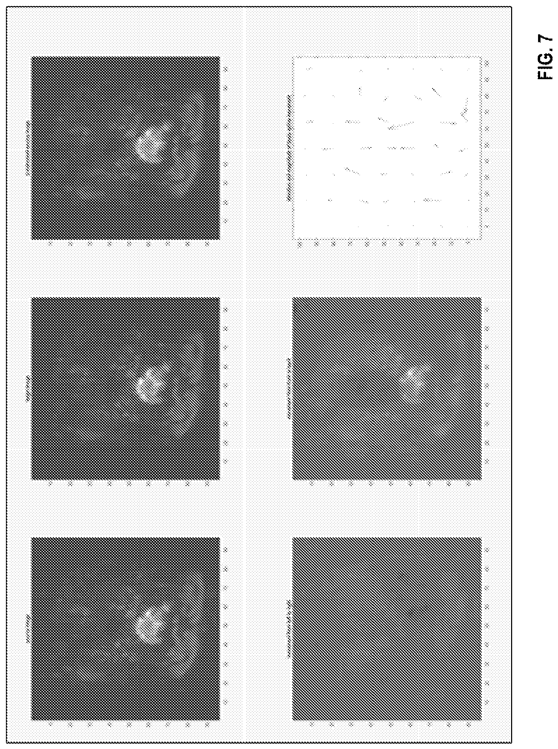

**FIG. 7** shows an exemplary nonlinear transform of fMRI images using nonlinear basis splines. A grid of 7x7 control points is distributed evenly on the source image, and each control point is positioned to minimize the difference between the transformed source and target. The optimum values are found using gradient descent, with a squared difference as the cost function. For example, a difference between a slice at Time 1 and a second slice from the same height at Time 2 may be determined. A grid of one or more control points can be overlaid on the slice, and each control point can independently apply X and Y translations to the data in the underlying slice. For example, the translation of each voxel in the slice may be interpolated between control points with bicubic interpolation. The density of the grid of one or more control points may be increased to arbitrary precision. By minimizing a cost function such as the squared difference between the two slices, the optimal position of each control point can be computed by gradient descent. When each control point of the one or more control points is weighted for its effect on the amount of T2* signal translated, a PCA of these weighted positions may be applied to obtain, for example, two eigenvectors which, in the absence of head motion, account for a large proportion of the variation in optimal control point location over the course of time. The eigenvalues of these weighted control point position components can be shown to correspond with deep and shallow breathing by comparing with a respiration belt or the variation in global signal strength from slice to slice. In the presence of head motion, rather than matching one slice directly to another, rotation parameters derived from the EEG can be used to fit the acquired T2* slice onto a previously acquired anatomical T2 whole brain volume, with the slice appropriately rotated and translated in space, although the gradient descent procedure could also allow the slice orientation and translation perpendicular to the slice plane to move relative to the T2 volume as additional parameters to be optimized. A representative weighted average of the higher resolution T2 image can be used to test goodness of fit to a lower resolution T2* fMRI slice voxel. The "best guess" temporal variation of cerebral blood flow may be iteratively incorporated in the model to minimize the error in translation induced by actual signal variation.

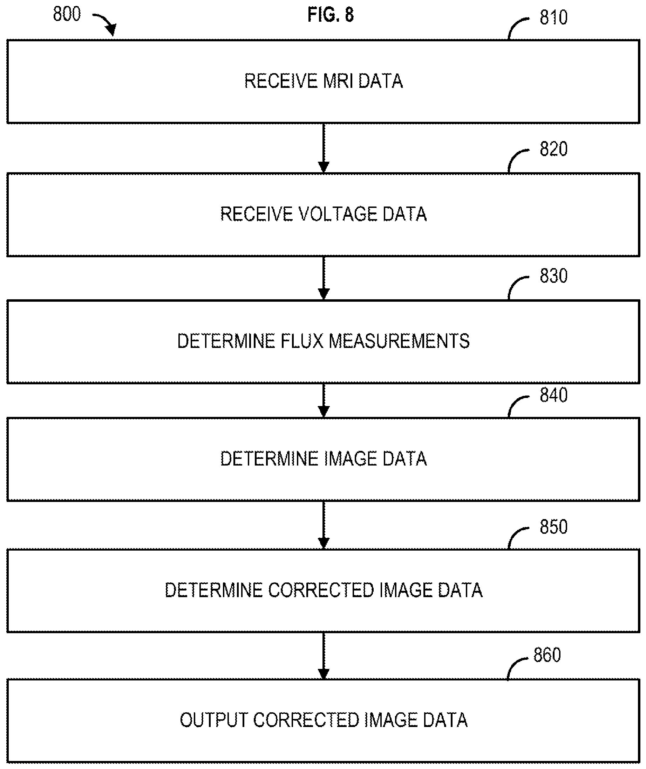

**FIG. 8** shows an example method. At step **810** MRI data may be received. The MRI image data may be determined by an MRI device and sent to a computing device. The MRI data may be determined, for example, by analyzing measurements and known or inferred perturbations of an electromagnetic field associated with an MRI procedure. The MRI data may comprise a plurality of voxels. The plurality of voxels may be associated with a respective plurality of regions in space. The plurality of voxels may be associated with a plurality of tensors. Each point in a tensor may be associated with a direction of a magnetic field at a point in space, of the strength of the magnetic field at the point in space, a rate of precession of the net magnetic field associated with a region in space, or a measured changed in magnetic flux associated with a region in space, or the like or combinations thereof. The MRI data may comprise a plurality of vectors associated with the plurality of voxels. The plurality of vectors associated with the plurality of voxels may indicate the strength and/or direction of an electromagnetic field at the plurality of points in space. The MRI data may include MRI timing data. The MRI timing information may be indicative of a change in the electromagnetic field over time at any point in space of the plurality of points in space. Analyzing the MRI data may comprise determining, for each image of a series of MRI images, magnetic field properties associated with points in a magnetic field and determining, for each image of the series of MRI images, a time stamp.

At **820,** voltage data may be received. The voltage data may comprise electroencephalography (EEG) data . The EEG data may comprise data related to electromagnetic signals detected by an EEG device. For example, the EEG data may comprise voltage data, current data, charge data, EEG image data, EEG timing data, and/or EEG error data. The voltage data may comprise data indicative of a position in space of a plurality of electrodes. For example the voltage data may indicate a combination of x, y, z coordinates. The voltage data may comprise measurements associated with the EEG timing data indicative of a magnetic field gradient. The voltage data may be associated with a location of at least one electrode of the plurality of electrodes. The voltage data may be associated with current flowing through a wire of plurality of wires associated with the plurality of electrodes. Determining the voltage data may comprise determining voltage field data. A position in space of one or more electrodes of a plurality of electrodes may be determined by measuring an electrical or magnetic property associated with each one of the plurality of electrodes as each one of the plurality of electrodes interacts with a magnetic field. In an embodiment, the data indicative of the position in space of the plurality of electrodes may be determined manually, that is to say, the position in space of each one of the plurality of electrodes may be determined in relation every other electrode of the plurality of electrodes, and not necessarily by measuring an electrical or magnetic property associated with each one of the plurality of electrodes as each one of the plurality of electrodes interacts with a magnetic field. The EEG image data may comprise a plurality of voxels. The plurality of voxels may be associated with a respective plurality of points in space. The EEG data may comprise a plurality of vectors associated with the plurality of voxels. The plurality of vectors associated with the plurality of voxels may indicate the strength and/or direction of an electromagnetic field at the plurality of points in space. The EEG data may comprise EEG timing data. The EEG timing may be indicative of a change in the electromagnetic field over time at any point in space of the plurality of points in space. Determining the EEG data may comprise determining a spatial location of a plurality of electrodes. Determining the EEG image data and the EEG timing data may comprise assigning, to each image of a series of EEG images, a time stamp.

At step **830,** one or more flux measurements may be determined. Based on the laws of electrodynamics, a voxel of the MRI magnetic field will impart more flux on an EEG wire when the MRI magnetic fields are closer to the wire (inversely proportional to the cube of the distance), more aligned with one another, precessing more quickly due to stronger gradients, or aligned in plane with the wire, having more surface area through which magnetic field lines can pass. The apparent scrambling of the MRI magnetic field achieved by a dephasing gradient reduces an MRI magnetic field voxel's alignment with its neighbors, which explains the rapid reduction in flux measured by the EEG. Further, it may be determined that, based on induced currents being in opposition to a reference electrode, slower precession is associated with an apparent "negative" voltage. The computing device **101** may be configured to determine one or more vectors (e.g., an eigenvector, a tensor, etc., combinations thereof and the like). For example, the computing device **101** may determine an electric current associated with the EEG device **102.** The electric current associated with the EEG device **102** may alternate it's current between a phase-encoding direction (e.g., front to back) and a frequency encoding direction (e.g., left to right). Thus, based on a model of the head of the patient (whether spherical or any other shape), the computing device **101** may determine a special organization vector (e.g., x, y, z, flux). The spatial organization vector may comprise a voxel. The spatial organization voxel may be determined across a time domain for every EEG sample at each EEG electrode.

At step **840,** image data may be determined. The image data may comprise a map of oxygenation in the brain and/or voltage signals. The image data may comprise skew distortions and/or scaling distortions as described above. The image data may comprise both the EEG image data and the MRI image data. The image data may be sequenced according to time. For instance, the image data may comprise a plurality of image data segments. Each of the image data segments may be associated with, for example, a slice, as described herein. Each of the slices may be time stamped. The time stamp may be determined according to the MRI data as the MRI is conducted and a series of scans are determined.

At step **850,** corrected image data may be determined. The corrected image data may be associated with at least one artifact in the EEG image data, the MRI data, and/or the combined data. Determining the corrected error data may comprise determining a correspondence between the EEG electrode location data and the measured voltage data during a known time when gradient magnetic fields are applied. Determining the corrected image data may comprise determining, using a model based on patient respiration, or by a spatially distributed array of magnetic field sensors in fixed locations, variation in time of the applied magnetic field gradient. Determining the corrected image data may comprise determining, based on an integrated effect of factors that are determined to repeat from a first excitation of a brain volume to either a second excitation of the brain volume or a plurality of brain volume excitations, an EEG gradient artifact wherein the EEG gradient artifact is associated with a variation in electrode location. Determining the corrected image data may comprise determining, based on a physiologically plausible waveform fit to the voltage data and varied using measured and estimated parameters that are known to affect cardiac output, an EEG ballistocardiogram artifact. Determining the corrected image data may comprise comparing, based on electrode location data, a first slice of MRI data associated with a first time stamp to a second slice of MRI data associated with a second time stamp, wherein the first slice is derived from a brain volume associated with the first time stamp and the second slice is derived from a brain volume associated with the second time stamp. Determining the corrected image data may comprise determining an optimal nonlinear transformation of each slice to match a corresponding slice or volume, and determining a functional magnetic resonance image respiration-induced susceptibility artifact associated with factors in an optimal transformation whose effects on a source image correspond with measured or computed respiration.

At **860,** the corrected image data may be output. Outputting the corrected image data may comprise sending the corrected image data to a remote device such as a display device.

The method may comprise synchronizing the MRI data and the voltage data according to the EEG timing data and the MRI timing data. Synchronizing the EEG data and the MRI data may comprise determining, for a given image of a plurality of EEG images timing information and determining, for a given image of a plurality of MRI images timing information and associating, based on the timing information of the respective images, the respective EEG and MRI images. The method may comprise performing a transformation on the combined image data and reducing, based on the transformation, imaging error.

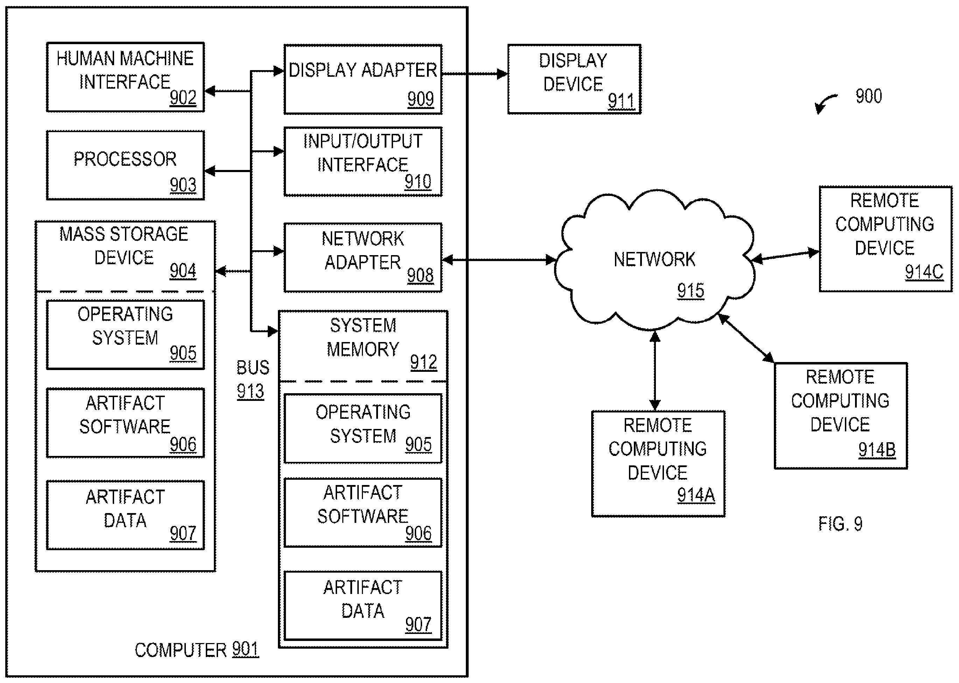

**FIG. 9** shows a system **900** for error correction in accordance with the present description. The computer **901** may comprise one or more processors **903,** a system memory **912,** and a bus **913** that couples various system components including the one or more processors **903** to the system memory **912.** In the case of multiple processors **903,** the computer **901** may utilize parallel computing. The bus **913** is one or more of several possible types of bus structures, including a memory bus or memory controller, a peripheral bus, an accelerated graphics port, or local bus using any of a variety of bus architectures.

The computer **901** may operate on and/or comprise a variety of computer readable media (e.g., non-transitory media). The readable media may be any available media that is accessible by the computer **901** and may include both volatile and non-volatile media, removable and non-removable media. The system memory **912** has computer readable media in the form of volatile memory, such as random access memory (RAM), and/or non-volatile memory, such as read only memory (ROM). The system memory **912** may store data such as artifact data **907** and/or program modules such as the operating system **905** and artifact software **906** that are accessible to and/or are operated on by the one or more processors **903.** The artifact data **907** may include, for example, one or more hardware parameters and/or usage parameters as described herein. The artifact software **906** may be used by the computer **901** to cause one or operations.

The computer **901** may also have other removable/non-removable, volatile/non-volatile computer storage media. **FIG. 9** shows the mass storage device **904** which may provide non-volatile storage of computer code, computer readable instructions, data structures, program modules, and other data for the computer **901.** The mass storage device **904** may be a hard disk, a removable magnetic disk, a removable optical disk, magnetic cassettes or other magnetic storage devices, flash memory cards, CD-ROM, digital versatile disks (DVD) or other optical storage, random access memories (RAM), read only memories (ROM), electrically erasable programmable read-only memory (EEPROM), and the like.

Any number of program modules may be stored on the mass storage device **904,** such as the operating system **905** and the artifact software **906.** Each of the operating system **905** and the artifact software **906** (e.g., or some combination thereof) may have elements of the program modules and the artifact software **906.** The artifact data **907** may also be stored on the mass storage device **904.** The image data **909** may be stored in any of one or more databases known in the art. Such databases may be DB2^{®}, Microsoft^{®} Access, Microsoft^{®} SQL Server. Oracle^{®}, mySQL, PostgreSQL, and the like. The databases may be centralized or distributed across locations within the network **915.**

A user may enter commands and information into the computer **901** via an input device (not shown). Examples of such input devices comprise, but are not limited to, a keyboard, pointing device (e.g., a computer mouse, remote control), a microphone, a joystick, a scanner, tactile input devices such as gloves, and other body coverings, motion sensor, and the like. These and other input devices may be connected to the one or more processors **903** via a human machine interface **902** that is coupled to the bus **913,** but may be connected by other interface and bus structures, such as a parallel port, game port, an IEEE 1399 Port (also known as a Firewire port), a serial port, network adapter **919,** and/or a universal serial bus (USB).

The display device **911** may also be connected to the bus **913** via an interface, such as the display adapter **909.** It is contemplated that the computer **901** may have more than one display adapter **909** and the computer **901** may have more than one display device **911.** The display device **911** may be a monitor, an LCD (Liquid Crystal Display), light emitting diode (LED) display, television, smart lens, smart glass, and/or a projector. In addition to the display device **911,** other output peripheral devices may be components such as speakers (not shown) and a printer (not shown) which may be connected to the computer **901** via the Input/Output Interface **910.** Any step and/or result of the methods may be output (or caused to be output) in any form to an output device. Such output may be any form of visual representation, including, but not limited to, textual, graphical, animation, audio, tactile, and the like. The display device **911** and computer **901** may be part of one device, or separate devices.

The computer **901** may operate in a networked environment using logical connections to one or more devices such as a personal computer, computing station (e.g., workstation), portable computer (e.g., laptop, mobile phone, tablet device), smart device (e.g., smartphone, smart watch, activity tracker, smart apparel, smart accessory), security and/or monitoring device, a server, a router, a network computer, a peer device, edge device, and so on. Logical connections between the computer **901** one or more devices may be made via a network **915,** such as a local area network (LAN) and/or a general wide area network (WAN). Such network connections may be through the network adapter **919.** The network adapter **919** may be implemented in both wired and wireless environments. Such networking environments are conventional and commonplace in dwellings, offices, enterprise-wide computer networks, intranets, and the Internet.

Application programs and other executable program components such as the operating system **905** are shown herein as discrete blocks, although it is recognized that such programs and components reside at various times in different storage components of the computing device **901,** and are executed by the one or more processors **903** of the computer. An implementation of the image software **906** may be stored on or sent across some form of computer readable media. Any of the described methods may be performed by processor-executable instructions embodied on computer readable media.

While specific configurations have been described, it is not intended that the scope be limited to the particular configurations set forth, as the configurations herein are intended in all respects to be possible configurations rather than restrictive. Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is in no way intended that an order be inferred, in any respect. This holds for any possible non-express basis for interpretation, including: matters of logic with respect to arrangement of steps or operational flow; plain meaning derived from grammatical organization or punctuation; the number or type of configurations described in the specification.

It will be apparent to those skilled in the art that various modifications and variations may be made without departing from the scope of the invention as claimed. Other configurations will be apparent to those skilled in the art from consideration of the specification and practice described herein. It is intended that the specification and described configurations be considered as exemplary only, with a true scope being indicated by the following claims.

## Claims

1. A computer-implemented method comprising:
receiving magnetic resonance imaging (MRI) data (810), wherein the MRI data comprises magnetic field data associated with a magnetic field;
receiving voltage data (802) from an electroencephalography (EEG) device (102), wherein the voltage data comprises data sample via one or more voltages from a plurality of electrodes in a preconfigured spatial configuration, wherein each electrode of the plurality of electrodes is associated with a position and a flux;
**characterized in that** the method further comprises:
determining, based on the MRI data and the voltage data, one or more flux measurements (830) wherein each flux measurement of the one or more flux measurements is associated with an electrode of the plurality of electrodes;
determining, based on the MRI data and the voltage data, first image data (840);
determining, based on the one or more flux measurements, corrected image data (850); and
outputting the corrected image data (860).

2. The computer-implemented method of claim 1, wherein receiving the MRI data comprises receiving the MRI data from an MRI device (103) and wherein the MRI data comprises one or more magnetic field properties associated with a magnetic field generated by the MRI device and wherein the MRI data comprises a series of images wherein each image of the series of images is associated with a time stamp.

3. The computer-implemented method of claim 1, further comprising:
determining, based on synchronized MRI timing data and voltage timing data, estimated MRI artifact data and estimated artifact voltage image data; and
determining, based on the synchronized MRI timing data and the voltage timing data, a match between a time stamp assigned to each image of a series of voltage images and a time stamp assigned to each image of a series of MRI images.

4. The computer-implemented method of any preceding claim, wherein the voltage data comprises measurements associated with the voltage timing data indicative of a magnetic field gradient.

5. The computer-implemented method of any preceding claim, further comprising determining voxel data, wherein the voxel data comprises a plurality of voxels and wherein each voxel of the plurality of voxels is associated with a plurality of tensors and wherein each tensor of the plurality of tensors is associated with an interaction between an electromagnetic field and one or more particles in a human brain.

6. The computer-implemented method of any of claims 1 or 5, wherein a first tensor is associated with a direction of a magnetic field at a point in space, and wherein a second tensor of the plurality of tensors is associated with a magnitude of the magnetic field at a point in space, and wherein a third tensor of the plurality of tensors is associated with direction and magnitude of a net magnetic field at a point in space, and wherein a fourth tensor of the plurality of tensors is associated with a rate of precession of the first tensor, and wherein a fifth tensor of the plurality of tensors is associated with a net change in magnetic flux influenced by a rate of precession of the first tensor.

7. The computer-implemented method of any preceding claim, wherein outputting the corrected image data comprises sending the corrected image data to a display device (911).

8. The computer-implemented method of any preceding claim, wherein determining the corrected image data comprises determining and removing one or more changes in one or more alignments of one or more normalized spatial vectors over time.

9. The computer-implemented method of any preceding claim, further comprising determining one or more coordinates associated with each electrode of the plurality of electrodes.

10. One or more non-transitory computer-readable media storing processor-executable instructions thereon that, when executed by a processor, cause the processor to perform the computer-implemented methods of any of claims 1-9.

11. A data processing system comprising:
a computing device (101) comprising means for carrying out the steps of the computer-implemented methods of any of claims 1-9; and
a display device (911) configured to output the corrected image data.

## Patentansprüche

1. Ein computerimplementiertes Verfahren, das umfasst:
Empfangen von Magnetresonanztomographie-Daten (MRI-Daten) (810), wobei die MRI-Daten Magnetfelddaten umfassen, die einem Magnetfeld zugeordnet sind;
Empfangen von Spannungsdaten (802) von einem Elektroenzephalografie-Gerät (EEG) (102), wobei die Spannungsdaten Datenproben über eine oder mehrere Spannungen von einer Vielzahl von Elektroden in einer vorkonfigurierten räumlichen Anordnung umfassen, wobei jede Elektrode der Vielzahl von Elektroden einer Position und einem Fluss zugeordnet ist;
**dadurch gekennzeichnet, dass** das Verfahren ferner umfasst:
Bestimmen einer oder mehrerer Flussmessungen (830) auf der Grundlage der MRT-Daten und der Spannungsdaten, wobei jede Flussmessung der einen oder mehreren Flussmessungen einer Elektrode der Vielzahl von Elektroden zugeordnet ist;
Ermitteln erster Bilddaten (840) auf der Grundlage der MRT-Daten und der Spannungsdaten;
Ermitteln korrigierter Bilddaten (850) auf der Grundlage der einen oder mehreren Flussmessungen; und
Ausgeben der korrigierten Bilddaten (860).

2. Das computerimplementierte Verfahren nach Anspruch 1, wobei das Empfangen der MRT-Daten das Empfangen der MRT-Daten von einer MRT-Vorrichtung (103) umfasst und wobei die MRT-Daten eine oder mehrere Magnetfeldeigenschaften umfassen, die einem von der MRT-Vorrichtung erzeugten Magnetfeld zugeordnet sind, und wobei die MRT-Daten eine Bildserie umfassen, wobei jedes Bild der Bildserie einem Zeitstempel zugeordnet ist.

3. Das computerimplementierte Verfahren nach Anspruch 1, das ferner umfasst:
Ermitteln von geschätzten MRT-Artefaktdaten und geschätzten Artefakt-Spannungsbilddaten auf der Grundlage von synchronisierten MRT-Zeitdaten und Spannungszeitdaten; und
Bestimmen, basierend auf den synchronisierten MRT-Zeitdaten und den Spannungszeitdaten, einer Übereinstimmung zwischen einem Zeitstempel, der jedem Bild einer Reihe von Spannungsbildern zugeordnet ist, und einem Zeitstempel, der jedem Bild einer Reihe von MRT-Bildern zugeordnet ist.

4. Das computerimplementierte Verfahren nach einem der vorstehenden Ansprüche, wobei die Spannungsdaten Messungen umfassen, die mit den Spannungszeitdaten assoziiert sind, welche einen Magnetfeldgradienten anzeigen.

5. Das computerimplementierte Verfahren nach einem der vorstehenden Ansprüche, das ferner das Bestimmen von Voxeldaten umfasst, wobei die Voxeldaten eine Vielzahl von Voxeln umfassen und wobei jedes Voxel der Vielzahl von Voxeln einer Vielzahl von Tensoren zugeordnet ist und wobei jeder Tensor der Vielzahl von Tensoren einer Wechselwirkung zwischen einem elektromagnetischen Feld und einem oder mehreren Partikeln in einem menschlichen Gehirn zugeordnet ist.

6. Das computerimplementierte Verfahren nach einem der Ansprüche 1 oder 5, wobei ein erster Tensor einer Richtung eines Magnetfelds an einem Punkt im Raum zugeordnet ist, und wobei ein zweiter Tensor der Vielzahl von Tensoren einer Stärke des Magnetfelds an einem Punkt im Raum zugeordnet ist, und wobei ein dritter Tensor der Vielzahl von Tensoren einer Richtung und einer Stärke eines Netto-Magnetfelds an einem Punkt im Raum zugeordnet ist, und wobei ein vierter Tensor der Vielzahl von Tensoren mit einer Präzessionsrate des ersten Tensors assoziiert ist, und wobei ein fünfter Tensor der Vielzahl von Tensoren mit einer Nettoänderung des Magnetflusses assoziiert ist, die durch eine Präzessionsrate des ersten Tensors beeinflusst wird.

7. Das computerimplementierte Verfahren nach einem der vorstehenden Ansprüche, wobei das Ausgeben der korrigierten Bilddaten das Senden der korrigierten Bilddaten an eine Anzeigevorrichtung (911) umfasst.

8. Das computerimplementierte Verfahren nach einem der vorstehenden Ansprüche, wobei das Bestimmen der korrigierten Bilddaten das Bestimmen und Entfernen einer oder mehrerer Änderungen in einer oder mehreren Ausrichtungen eines oder mehrerer normalisierter Raumvektoren im Zeitverlauf umfasst.

9. Das computerimplementierte Verfahren nach einem der vorstehenden Ansprüche, das ferner das Bestimmen einer oder mehrerer Koordinaten umfasst, die jeder Elektrode der Vielzahl von Elektroden zugeordnet sind.

10. Ein oder mehrere nichtflüchtige, computerlesbare Medien, auf denen prozessorausführbare, Befehle gespeichert sind, die, wenn sie von einem Prozessor ausgeführt werden, den Prozessor veranlassen, die computerimplementierten Verfahren gemäß einem der Ansprüche 1 bis 9 auszuführen.

11. Ein Datenverarbeitungssystem, das umfasst:
eine Recheneinrichtung (101), die Mittel zum Ausführen der Schritte der computerimplementierten Verfahren gemäß einem der Ansprüche 1 bis 9 umfasst; und
eine Anzeigevorrichtung (911), die so konfiguriert ist, dass sie die korrigierten Bilddaten ausgibt.

## Revendications

1. Procédé mis en œuvre par ordinateur, comprenant :
la réception de données d'imagerie par résonance magnétique (IRM) (810), les données IRM comprenant des données de champ magnétique associées à un champ magnétique ;
la réception de données de tension (802) provenant d'un dispositif d'électroencéphalographie (EEG) (102), les données de tension comprenant des échantillons de données via une ou plusieurs tensions provenant d'une pluralité d'électrodes disposées selon une configuration spatiale préconfigurée, chaque électrode de la pluralité d'électrodes étant associée à une position et à un flux ;
**caractérisé en ce que** le procédé comprend en outre :
déterminer, sur la base des données IRM et des données de tension, une ou plusieurs mesures de flux (830), dans lequel chaque mesure de flux parmi lesdites une ou plusieurs mesures de flux est associée à une électrode de la pluralité d'électrodes ;
la détermination, sur la base des données IRM et des données de tension, de premières données d'image (840) ;
déterminer, sur la base de la ou des mesures de flux, des données d'image corrigées (850) ; et
la sortie des données d'image corrigées (860).

2. Procédé mis en œuvre par ordinateur selon la revendication 1, dans lequel la réception des données IRM comprend la réception des données IRM provenant d'un dispositif IRM (103) et dans lequel les données IRM comprennent une ou plusieurs propriétés de champ magnétique associées à un champ magnétique généré par le dispositif IRM et dans lequel les données IRM comprennent une série d'images, chaque image de la série d'images étant associée à un horodatage.

3. Procédé mis en œuvre par ordinateur selon la revendication 1, comprenant en outre :
la détermination, sur la base de données de synchronisation IRM et de données de synchronisation de tension synchronisées, de données d'artefacts IRM estimées et de données d'images de tension d'artefacts estimées ; et
déterminer, sur la base des données de synchronisation IRM et des données de synchronisation de tension, une correspondance entre un horodatage attribué à chaque image d'une série d'images de tension et un horodatage attribué à chaque image d'une série d'images IRM.

4. Procédé mis en œuvre par ordinateur selon l'une quelconque des revendications précédentes, dans lequel les données de tension comprennent des mesures associées aux données de synchronisation de tension indicatives d'un gradient de champ magnétique.

5. Procédé mis en œuvre par ordinateur selon l'une quelconque des revendications précédentes, comprenant en outre la détermination de données de voxels, dans lequel les données de voxels comprennent une pluralité de voxels et dans lequel chaque voxel de la pluralité de voxels est associé à une pluralité de tenseurs et dans lequel chaque tenseur de la pluralité de tenseurs est associé à une interaction entre un champ électromagnétique et une ou plusieurs particules dans un cerveau humain.

6. Procédé mis en œuvre par ordinateur selon l'une quelconque des revendications 1 ou 5, dans lequel un premier tenseur est associé à une direction d'un champ magnétique en un point de l'espace, et dans lequel un deuxième tenseur de la pluralité de tenseurs est associé à une amplitude du champ magnétique en un point de l'espace, et dans lequel un troisième tenseur de la pluralité de tenseurs est associé à la direction et à l'amplitude d'un champ magnétique net en un point de l'espace, et dans lequel un quatrième tenseur de la pluralité de tenseurs est associé à une vitesse de précession du premier tenseur, et dans lequel un cinquième tenseur de la pluralité de tenseurs est associé à une variation nette du flux magnétique influencée par une vitesse de précession du premier tenseur.

7. Procédé mis en œuvre par ordinateur selon l'une quelconque des revendications précédentes, dans lequel la sortie des données d'image corrigées comprend l'envoi des données d'image corrigées à un dispositif d'affichage (911).

8. Procédé mis en œuvre par ordinateur selon l'une quelconque des revendications précédentes, dans lequel la détermination des données d'image corrigées comprend la détermination et la suppression d'un ou plusieurs changements dans un ou plusieurs alignements d'un ou plusieurs vecteurs spatiaux normalisés au fil du temps.

9. Procédé mis en œuvre par ordinateur selon l'une quelconque des revendications précédentes, comprenant en outre la détermination d'une ou plusieurs coordonnées associées à chacune des électrodes de la pluralité d'électrodes.

10. Un ou plusieurs supports non transitoires lisibles par ordinateur stockant des instructions d' s exécutables par un processeur qui, lorsqu'elles sont exécutées par un processeur, amènent le processeur à mettre en œuvre les procédés mis en œuvre par ordinateur selon l'une quelconque des revendications 1 à 9.

11. Système de traitement de données comprenant :
un dispositif informatique (101) comprenant des moyens pour exécuter les étapes des procédés mis en œuvre par ordinateur selon l'une quelconque des revendications 1 à 9 ; et
un dispositif d'affichage (911) configuré pour fournir en sortie les données d'image corrigées.
